# EUROPÄISCHE PATENTSCHRIFT

(11) **EP 1 393 044 B1**
(45) Veröffentlichungstag und Bekanntmachung des Hinweises auf die Patenterteilung: **14.07.2010**
(21) Anmeldenummer: 02740461.5
(22) Anmeldetag: 11.04.2002
(51) Int. Cl.: G01N 15/08, G01M 3/32

(54) **VERFAHREN UND VORRICHTUNG ZUM BESTIMMEN DER PERMEATION EINER BARRIERESCHICHT**
METHOD AND DEVICE FOR DETERMINING THE PERMEATION OF A BARRIER LAYER
PROCEDE ET DISPOSITIF POUR DETERMINER LA PERMEABILITE D'UNE COUCHE BARRIERE

(30) Priorität: 18.05.2001 DE 10124225
(43) Veröffentlichungstag der Anmeldung: 03.03.2004
(73) Patentinhaber: Tetra Laval Holdings & Finance SA, 1009 Pully (CH)
(72) Erfinder: MOORE, Rodney, 64342 Seeheim-Jugenheim (DE)
(74) Vertreter: Weber, Roland
(86) Internationale Anmeldenummer: PCT/EP2002/004016
(87) Internationale Veröffentlichungsnummer: WO 2002/095369

(56) Entgegenhaltungen:
- EP-A- 0 833 139
- WO-A-01/48452
- CA-A- 2 122 250
- US-A- 5 591 898

## Beschreibung

Die Erfindung betrifft ein Verfahren und eine Vorrichtung zum Bestimmen der Permeation einer Barriereschicht auf der Oberfläche eines Behälters, bei welchem der Behälter in ein druckdicht abschließbares Gefäß verbracht wird, der Druck über der Zeit gemessen, die Steigung der Druck-Zeit-Kurve ermittelt wird und aus diesen Messdaten die Permeation der Barriereschicht bestimmt wird.

Es ist bekannt, Flüssigkeitspackungen aus Kunststoff herzustellen, zum Beispiel in Form von Flaschen mit oben befindlicher Öffnung. Solche Flaschen sind aus Polyethylenterephthalat (PET) hergestellt und werden vielfach für die Verpackung zum Beispiel von Wasser verwendet. Man weiß, dass die Wandungen der Kunststoffpackungen für niedermolekulare Gase durchlässig sind, weshalb die Haltbarkeitszeiten für flüssige Lebensmittel, insbesondere Säfte und CO₂-haltige Mischgetränke, begrenzt sind. Um den dafür verantwortlichen Sauerstoff am Eindringen bzw. das Kohlendioxid am Entweichen zu hindern und damit solche Kunststoffverpackungen für flüssige Lebensmittel besser anwendbar zu machen, ist man dazu übergegangen, die Wandungen dieser Kunststoffpackungen mit Barriereschichten zu versehen, zum Beispiel auf der inneren Oberfläche von Kunststoffflaschen.

Es versteht sich daher, dass man bei der Verwendung von Kunststoffbehältern, insbesondere solchen aus PET, auf eine möglichst hohe Barrierewirkung der Wandung achten muss. Es sind deshalb bereits Verfahren zur Messung der Barriereeigenschaften einer Kunststoffwandung entwickelt worden. Bei einem solchen bekannten Verfahren sättigt man zum Beispiel eine zu messende, als Barriere wirkende Behälterwandung mit einem Testgas und lässt dann nach dem Sättigen die in die Behälterwandung bzw. Materialprobe eingeladenen Stoffe in ein druckdicht abschließbares Gefäß ausgasen. Dadurch steigt in diesem Gefäß der betreffende Partialdruck und kann in bestimmten zeitlichen Abständen gemessen werden. Es ergibt sich eine Druck-Zeit-Kurve, aus der man die Permeation bzw. Durchlässigkeit des zu untersuchenden Materials mit seiner Barriereschicht berechnen kann.

Mit Nachteil dauert das Beladen, Entgasen und Messen bis zu zwei Stunden, so dass dieses Bestimmungsverfahren für eine industrielle Anwendung bei der Herstellung von Kunststoffverpackungen mit großer Leistung (große Stückzahl pro Zeiteinheit) nicht geeignet ist.

Aus der EP-A-0833139 ist ein Verfahren zur Lecksuche und zur Messung von beschichteten Behältern bekannt, wobei eine Druck-Zeit-Kurve erstellt wird. Die beschichteten Behälter sind Vakuumröhrchen, in denen Patientenblut gesammelt wird und bei denen es auf die Haltbarkeitsdauer ankommt. Das zuvor und getrennt evakuierte Teströhrchen wird in ein druckdichtabschließbares Gefäß gebracht, dessen Druck über Umgebungsdruck so angehoben wird, dass bei schnellem Druckanstieg in dem Gefäß eine Permeation seines Gases durch die beschichtete Wand des Behälters bzw. Teströhrchens beginnt. Für die Druck-Zeit-Kurve wird der Druck in dem abschließbaren Gefäß als Druckveränderung gegenüber einem abgeschlossenen Referenzgefäß gemessen. Es wird also die Gasleckage in das Blutsammelröhrchen hinein als Differenzdruck gemessen. Die Quantifizierung des Gastransportes erfolgt in zwei bis zehn Tagen. Das bekannte Verfahren und die dafür eingerichtete Vorrichtung eignen sich nicht für eine industrielle Anwendung in dem vorstehend beschriebenen Sinne.

Es ist daher Aufgabe der vorliegenden Erfindung, ein Bestimmungsverfahren der eingangs bezeichneten Art dahingehend weiter zu verbessern, dass für industrielle Anwendung die Permeation einer Vielzahl von Behältern nacheinander in jeweils kürzerer Zeit als in 1-1 1/2 Stunden und unter Einsatz einfacherer Messgeräte bestimmt werden kann.

Das Verfahren und die Vorrichtung gemäss der Erfindung haben sich die industrielle Anwendung zum Ziel gesetzt, wobei die Permeation der betreffenden Barriereschicht nicht derart aufwendig gemessen werden muss, dass das Messergebnis hochqualifizierten Eichbedingungen genügt. Vielmehr reicht es, die Permeation der Schicht in dem jeweiligen Behälter so zu bestimmen, dass man sie als gut, ausreichend oder mangelhaft bezeichnen kann bzw. muss.

Diese Aufgabe wird erfindungsgemäß für das Verfahren dadurch gelöst, dass das druckdicht abschließbare Gefäß und der darin befindliche Behälter evakuiert werden, nach einer Konditionierungszeit der Behälter auf der Seite mit der Barriereschicht entgasen gelassen wird und der Partialdruck wenigstens eines aus der Behälterwandung abgegebenen Stoffes, insbesondere Gases, gemessen wird. Das erfindungsgemäße Verfahren lässt sich insbesondere für Barriereschichten verwenden, welche sich auf der inneren Oberfläche eines Behälters befinden, zum Beispiel einer Innenseite einer Verpackung. Besonderes Augenmerk bei der Durchführung des Verfahrens wird dabei auf Verpackungen aus Kunststoff, insbesondere in Form einer oben offenen Flasche gerichtet. Bei einem solchen flaschenförmigen Behälter kann man durch einen Stopfen sein Inneres druckdicht verschließen. Da der Behälter in dem druckdicht abschließbaren Gefäß untergebracht wird, kann man den Druck innerhalb und außerhalb des Behälters und damit auf beiden Seiten der Barriereschicht beeinflussen, einstellen und messen.

Es ist bevorzugt, den zu untersuchenden Behälter außerhalb des abschließbaren Gefäßes und damit vor Beginn des Verfahrens zu konditionieren. Wie in bekannten Fällen wird die Wandung des zu untersuchenden Behälters eine bestimmte Zeit lang mit bestimmten Gasen beladen. Bei dem Einsatz des erfindungsgemäßen Verfahrens an Herstellungsmaschinen mit großer Leistung ist es insbesondere für industrielle Anwendung zweckmäßig, wenn man die ohnehin notwendige Lagerzeit fertiger Behälter und die diese umgebende Raumluft bei Zimmertemperatur zum Konditionieren verwendet. Außerdem kann es günstig sein, unter Konditionieren auch den weiteren Schritt einer anfänglichen Entgasung zu verstehen, um nämlich beim Ermitteln der Druck-Zeit-Kurve in ähnliche oder vorzugsweise sogar die gleichen Druckbereiche zu kommen, in welchen dann die Zeit gemessen wird, innerhalb welcher der Druck sich im Behälter durch das Entgasen auf den vorbestimmten Enddruck steigert. Dieses Vorentgasen gelingt in kurzer Zeit und begünstigt die Messung erheblich.

Die erwähnten Merkmale des erfindungsgemäßen Verfahrens reichen für das Messen des Partialdruckes nur eines, zweier oder dreier Stoffe aus. Ein solches Verfahren lässt sich zum Beispiel mittels eines Massenspektrometers durchführen, welches zwar selbst in einem Druckbereich der Größenordnung von etwa 10<-6> mbar misst, welches aber durch eine parallel angelegte Pumpe, vorzugsweise eine Turbomolekularpumpe und eine Vorpumpe, auch an Messleitungen angeschlossen werden kann, deren Druck im Bereich 1 bis 10 Millibar liegt. Mit einem Quadrupol-Messkopf erlaubt ein Massenspektrometer die Messung verschiedener Peaks mit unterschiedlichen atomaren Masseneinheiten. Hier interessieren besonders die Peaks für zum Beispiel Wasser, Stickstoff oder Sauerstoff. Das erfindungsgemäße Verfahren empfiehlt dann die Messung der Zeitdifferenz, innerhalb der die betreffende Linie (oft auch englisch als "Peak" bezeichnet) einen vorgewählten Zuwachs ihrer Intensität relativ zu einem bestimmten Anfangswert oder zu einer anderen Linie erreicht hat. Zwar hat man hier eine Intensitäts-Zeit-Kurve, die aber im Prinzip gleich der vorerwähnten Druck-Zeit-Kurve entspricht. Auch in dieser Intensitäts-Zeit-Kurve findet man lineare Bereiche. Damit kann man wieder aus der Zeitdauer auf die Qualität der Barriereschicht, nun aber für einen bestimmten Stoff schließen.

Auf dem Markt gibt es die unterschiedlichsten Vakuumpumpen, so dass das Evakuieren des zu untersuchenden Behälters einerseits (innen) als auch des ihn umschließenden Gefäßes andererseits (außen) in einfacher Weise und sehr schnell erfolgen kann. Es hat sich gezeigt, dass es erfindungsgemäß günstig ist, wenn man wenigstens einen der Räume auf etwa 1 Millibar Druck evakuiert. Das erste Vorentgasen, um den für die Reproduzierbarkeit des Bestimmungsverfahren erreichten Druckbereich zu erreichen, kann unter oder über dem Druckwert von 1 Millibar durchgeführt werden, je nach dem vorgewählten Druckbereich, in welchem die Druck-Zeit-Kurve als besonders relevant betrachtet wird.

Aus dieser Druck-Zeit-Kurve wird man sich zum Erreichen gut reproduzierbarer und für das Erreichen genauer Werte einen Abhängigkeitsbereich heraussuchen, bei welchem die Verhältnisse von Druck und Zeit im wesentlichen linear sind. Erfindungsgemäß sucht man sich aus der Druck-Zeit-Kurve also denjenigen (beschränkten) Bereich heraus, in welchem sich der Druck linear zur Zeit verändert. Die sich dann ergebende Steigung stellt ein direktes Maß für die Qualität der Barriereschicht dar. Wählt man zum Beispiel einen Druckbereich zwischen 3 und 4 mbar aus, dann bedeutet ein größeres Zeitintervall zum Erreichen dieses Druckunterschiedes zwischen Anfang und Ende beim Entgasen, dass die Qualität der Barriereschicht gut ist und umgekehrt. Dahinter steht die Modellvorstellung, dass die Kunststoffwandung des zu messenden Behälters zu Beginn des Bestimmungsverfahrens mit Gasen und eventuell auch Wasser aus der Umgebung bis zu einer gewissen Sättigung beladen ist. Bei einer Beschichtung der inneren Oberfläche des zu messenden Behälters mit einer Barriere verhindert letztere ein schnelles Ausgasen. Deshalb gibt die Zeit, innerhalb welcher der vorgewählte Druckanstieg erreicht ist, einen klaren Hinweis darauf, wie vollständig oder mangelhaft die Barriereschicht das Entgasen der Behälterwandung verhindert hat.

Weil die Behälter in aller Regel und insbesondere bei der industriellen Anwendung mit einem Stoffgemisch, unter anderem einem Gasgemisch, beladen werden, wird vorzugsweise bei Einsatz einfacher Messgeräte die Summe aller Partialdrücke zusammen gemessen, und der sich daraus ergebende Gesamtdruck kann mit vollem Erfolg für das Ermitteln der Steigung der Druck-Zeit-Kurve eingesetzt werden. Wenn für die geplante industrielle Anwendung die Summe der Partialdrücke aller aus der Behälterwandung abgegebener Stoffe gemessen wird, braucht also nicht auf einen speziellen Partialdruck besonders geachtet oder dieser artspezifisch behandelt zu werden, sondern man kann mit einer einfachen Standard-Vakuummessröhre, zum Beispiel einer Kapazitätsmessröhre, den Druck der entgasten Stoffe messen. Die Praxis hat gezeigt, dass eine solche Messröhre lediglich im Millibarbereich messen muss und andere, durch Zehnerpotenzen hiervon entfernte Messbereiche geringeren Drucks nicht abzudecken braucht.

Das erfindungsgemäße Bestimmungsverfahren benötigt nicht den Einsatz hoher Temperaturen, die möglicherweise Beschädigungen der Wandungsmaterialien des zu messenden Behälters hervorrufen. Es brauchen auch nicht chemische Stoffe eingesetzt zu werden, um die Barriereschicht oder die dahinter befindliche Behälterwandung zu beeinflussen und möglicherweise zu zerstören. In weniger als 10 Minuten, vorzugsweise weniger als 5 Minuten, kann das Erreichen der vorgewählten Druckdifferenz gemessen werden und über die bestimmte Steigung der Druck-Zeit-Kurve eine Aussage über die Qualität der Barriereeigenschaft getroffen werden. Je geringer die Steigung, d. h. je größer der Zeitraum zum Erreichen des oberen Druckendwertes ist, um so höher ist die Qualität der Barriereschicht, deren Beurteilung zwischen gut, ausreichend oder mangelhaft bereits genügt.

Im Zusammenhang mit der zuletzt erwähnten Ausführungsform des erfindungsgemäßen Bestimmungsverfahrens ist es bei weiterer vorteilhafter Ausgestaltung zweckmäßig, wenn der Partialdruck wenigstens eines von der Behälterwandung abgegebenen, für die geschmackliche Beeinflussung der Behälterfüllung relevanten Stoffes gemessen wird. Der Endverbraucher kann bereits derzeit mehr oder weniger stark mit CO2 versetzte Sprudelwasser in Kunststoffflaschen kaufen, welche den Glasflaschen ähnlich sind, unter anderem aber demgegenüber den Vorteil des geringeren Gewichtes haben. Bei schlechter Verarbeitung kennt der Endverbraucher den unangenehmen "Kunststoff- Geschmack", wenn das aus einer solchen Flasche entleerte Wasser nach "Kunststoff" schmeckt. Inzwischen wurde untersucht und festgestellt, welche Stoffe für diese Geschmacksgefährdung relevant sind. Diese Stoffe sind bekannt und können mit dem vorerwähnten Massenspektrometer detektiert werden. Ein bekannter Vertreter solcher Stoffe ist Acetaldehyd. Die Messung des Partialdruckes eines solchen geschmacklich relevanten Stoffes in einem Füllgut, zum Beispiel Sprudelwasser, erlaubt das Bestimmen der Barrierequalität. Wird nämlich eine solche Sprudelflasche aus Kunststoff auf ihrer inneren Oberfläche mit einer Barriereschicht zum Beispiel auf Quarzbasis versehen, dann gelingt es bisweilen, geschmacksrelevante, in der Kunststoffwandung des Behälters befindliche Stoffe am Austreten in das Füllgut zu hindern. Der Grad der Verhinderung soll selbstverständlich möglichst groß sein und kann mit dem erfindungsgemäßen Verfahren der eben beschriebenen Art festgestellt werden.

Als besonders vorteilhaft hat man das erfindungsgemäße Verfahren beurteilt, wenn bei weiterer Ausgestaltung der Erfindung die Qualität der Barriereschicht eines Referenzbehälters zum Eichen der Steigung der gemessenen Druck-Zeit-Kurve verwendet wird. Es gibt sehr genaue Messverfahren für die Barrierequalität eines Behälters, allerdings mit dem Nachteil, dass 2 Stunden bis zu 5 Tage notwendig sind, um den exakten, geeichten Wert wissenschaftlich zu erfassen. Setzt man nun einen solchen bereits gemessenen Referenzbehälter bei der Durchführung des erfindungsgemäßen Verfahrens ein, dann zeigt dieses in der gemessenen Druck-Zeit-Kurve eine bestimmte Steigung, die beispielsweise für die Beurteilung "gut" steht. Erfindungsgemäß kann man nun die Qualität der Barriereschicht des zu messenden Behälters durch Vergleich mit dem Referenzbehälter bestimmen. Unter Weglassen zahlreicher Bedingungen und Merkmale wissenschaftlich exakter Messverfahren genügt es erfindungsgemäß, die in verhältnismäßig kurzer Zeit messbare Steigung der Druck-Zeit- Kurve zu erhalten und mit der eines exakt gemessenen Referenzbehälters zu vergleichen.

Das erfindungsgemäße Bestimmungsverfahren kann hinsichtlich Genauigkeit und Reproduzierbarkeit dadurch noch weiter beeinflusst werden, dass erfindungsgemäß in der Konditionierungszeit alle dem Bestimmungsverfahren zu unterziehenden Behälter gleichmäßig mit Molekülen beladen werden. Man weiß, dass der Sättigungsgrad und damit die Messergebnisse von der Vorbehandlung in der Konditionierungszeit abhängen. Daher ist man bestrebt, alle zu untersuchenden Behälter gleichmäßig vorzubehandeln. Im Sinne der oben erwähnten Modellvorstellung wird der Benutzer des Verfahrens die Wandungen der zu untersuchenden Behälter gleichmäßig mit den zur Verfügung stehenden Molekülen beladen, zum Beispiel sättigen.

Günstig ist es gemäss der Erfindung, wenn der Behälter und das druckdicht abschließbare Gefäß in etwa 1 Minute evakuiert werden. Praktische Versuche haben beim Einsatz einfacher Vakuumpumpen diesen Wert ergeben, der bei der industriellen Anwendung durchaus begrüßt wird. Dem steht nicht entgegen, dass man bei Einsatz stärkerer Vakuumpumpen das Evakuieren auch in kürzerer Zeit erreichen kann.

Dabei hat man ferner gefunden, dass es erfindungsgemäß günstig ist, wenn das Entgasen und Messen des wenigstens einen Partialdrucks insgesamt weniger als 8 Minuten, vorzugsweise 6 Minuten und ganz besonders bevorzugt etwa 4 Minuten dauern. Wenn das Volumen des Aufbaues zur Durchführung des erfindungsgemäßen Verfahrens festgelegt ist, können die vorgenannten Zeitwerte schon bei Einsatz einfacher Messgeräte erreicht werden. Nach dem etwa 1-minütigen Evakuieren kann es ausreichen, ein Entgasen etwa 2 Minuten lang durchzuführen, um sogar in höheren Bereichen der Druckdifferenz den ins Auge gefassten Messbereich der Druck-Zeit-Kurve zu finden, zum Beispiel den Messbereich zwischen 6 Millibar und 6,5 Millibar. Man hat hier gute Linearität zwischen Druck und Zeit gefunden und zufriedenstellende Messergebnisse erreicht. Gemessen wird die Zeitdifferenz, in welcher zum Beispiel der Druck von 6 Millibar auf 6,5 Millibar gestiegen ist. Diese Messung kann man innerhalb 1 Minute erreichen. Insgesamt ergibt sich also für das Evakuieren, das Entgasen in der Vorlaufzeit zum Erreichen des gewünschten Messbereiches und danach die Messzeit ein Gesamtzeitaufwand von etwa 4 Minuten. Dies ist ein überraschender Vorteil gegenüber der bisher notwendigen Zeit von etwa 2 Stunden oder etwas weniger.

Die oben erläuterte Qualitätsbestimmung der Barriereschicht erfolgte durch die Messung der Partialdrücke, nachdem das druckdicht abschließbare Gefäß und der darin befindliche Behälter evakuiert wurden. Es versteht sich dass für dieses Evakuieren einige Zeit erforderlich ist, bei erfolgreich durchgeführten Versuchen erfindungsgemäß 2 bis 3 Sekunden. Die oben beschriebene Messung der Partialdrücke erfolgt dann in dem erwähnten Bereich der Druck-Zeit-Kurve, in welchem sich der Druck linear zur Zeit verändert. Die sich ergebende Steigung stellt, wie oben erwähnt, ein direktes Maß für die Qualität der Barriereschicht dar. Dieses Messverfahren gilt sowohl für Barriereschichten, die sich auf der inneren Oberfläche als auch - bei anderen Ausführungsformen - auf der äußeren Oberfläche eines Behälters befinden.

Basierend auf der gleichem Modellvorstellung ist es überraschenderweise nach einer zusätzlichen Überlegung auch möglich, eine Art Vormessung oder Indikationsmessung durchzuführen, die auf dem gleichen erfindungsgemäßen Verfahren beruht. Bei der laufenden Produktion mit einer Hochleistungsmaschine können beispielsweise 10.000 PET-Flaschen pro Stunde produziert werden, wobei eine Barriereschicht auf der inneren Oberfläche der Flasche oder auch auf der äußeren Oberfläche derselben in einem Beschichtungsprozess aufgebracht wird. Es ist verständlich, dass unerwartet Produktionsfehler auftreten können, wobei z. B. einige produzierte PET-Flaschen (Behälter) durch eine fehlerhafte Beschichtungseinrichtung nicht mit einer Barriereschicht versehen sind. Durch die vorstehende Qualitätsmessung wird dies zwar festgestellt, den Beschichtungsprozess kann man aber schneller beeinflussen, wenn man etwaige Fehler schneller feststellt. Dies gelingt mit Vorteil, wenn man die Produkte, z. B. die PET-Flaschen, in Messkanälen gleich nach der Produktion untersucht.

Wenn nun nach einer weiteren vorteilhaften Ausgestaltung der Erfindung bei dem Evakuieren die Summe aller Partialdrücke des im Behälter oder außerhalb desselben befindlichen Gases und der aus der Behälterwandung abgegebenen Stoffe gemessen werden, kann dies in wesentlich kürzerer Zeit erfolgen. Diese Vormessung oder Indikationsmessung soll nicht die oben beschriebene Qualitätsmessung ersetzen. Sie kann diese aber mit großem Vorteil ergänzen. Zum Beispiel kann das Versagen einer Beschichtungsvorrichtung in wesentlich kürzerer Zeit erkannt werden. Man erhält dann in kürzerer Zeit nach der Produktion eine Indikation bzw. ein grobes Messergebnis, aus welchem man mit ausreichender Sicherheit feststellen kann, ob das Produkt, der betreffende beschichtete Behälter, mit einer Barriereschicht versehen ist, die den genaueren Qualitätsmessungen genügt.

Der Grundgedanke dieser erfindungsgemäßen Weiterentwicklung ist es, die Evakuierung des abschließbaren Gefäßes und des darin befindlichen Behälters bereits zu einer Messung zu benutzen bzw. eine Druckmessung in derjenigen Anfangszeit durchzuführen, in welcher das Entgasen oder Evakuieren erst vorgenommen wird. Nach dem eingangs schon erläuterten Modell wird die Kunststoffwandung des zu messenden Behälters mit Stoffen, insbesondere Gasen und eventuell auch Wasser aus der Umgebung beladen. Nach diesem Beladen erfolgt das Entgasen. Bei einer Beschichtung der inneren oder äußeren Oberfläche des zu messenden Behälters mit einer Barriere verhindert letztere bei ausreichender Qualität ein schnelles Ausgasen. Deshalb gibt auch schon während des Evakuierens bzw. Entgasens die Zeit, innerhalb welcher ein vorgewählter Evakuierungsgrad erreicht ist, einen klaren Hinweis darauf, ob die Barriereschicht bei ausreichender Qualität ein Entgasen der Behälterwandung verhindert hat.

Beim Evakuieren werden allerdings nicht nur die Partialdrücke der aus der Behälterwandung abgebebenen Stoffe gemessen, sondern auch die Partialdrücke desjenigen Gases, welches sich auf der Seite befindet, wo die Wandung beschicht ist. Ist eine PET-Flasche auf ihrer inneren Oberfläche beschichtet, dann werden auch die Partialdrücke des im Behälter befindlichen Gases gemessen. Befindet sich die Beschichtung auf der äußeren Oberfläche des Behälters, z. B. der PET-Flasche, dann werden auch die Partialdrücke der außerhalb der PET-Flasche befindlichen Gase gemessen. Dieses Messverfahren arbeitet einwandfrei, wenn man die Summe aller Partialdrücke der Gase misst. Für deren Messung, d.h. für die eigentliche Druckmessung, wird ein an das Gefäßvolumen angeschlossenes Druckmessgerät verwendet, z. B. eine Kapazitätsmessröhre.

Günstig ist es gemäß der Erfindung weiterhin, wenn die Partialdrücke nach einer festgesetzten Zeit gemessen und mit einem Zieldruckwert verglichen werden. Man beginnt mit der Durchführung des Verfahrens zu Beginn einer Duck-Zeit-Kurve und misst die Partialdrücke zu einer ähnlich einer Eichung festgesetzten Zeit. Dabei vergleicht man den Messwert der Partialdrücke mit einem Zieldruckwert, für den man auch einen gewissen Bereich ansetzen kann. Wenn die Messung nach der festgelegten Zeit einen Druckwert ergibt, der in der Nähe des Zielwertes liegt, dann schließt man auf eine ausreichende Qualität der Barriereschicht. Der Druckbereich liegt in der Größenordnung von 1/100 mbar.

Alternativ kann man erfindungsgemäß mit Vorteil auch die Zeit messen, in welcher ein vorgegebener Zielpartialdruck erreicht wird. Wir beispielsweise der Zielpartialdruck erst nach einer zu langen Zeit erreicht, dann kann man auf eine mangelhafte Qualität der Barriereschicht schließen.

Das neue Verfahren für die Vormessung oder Indikationsmessung mag zwar gegenüber dem vorgenannten Hauptverfahren beim Messen des Druckanstiegs mit Schwankungen behaftet sein, die bei durchgeführten Versuchen im Bereich von 20 % gegenüber der genauen Messung liegen. Dieser Streubereich ist aber unschädlich, weil man trotzdem klar einen Qualitätstrend feststellen kann. Dieser Streubereich ergibt sich z. B. durch Schwankungen der Leistung der Evakuierungspumpen. Deren Leistung schlägt zwar auf die Indikationsmessung durch. Man erreicht aber in erheblich kürzerer Zeit Vormessungen, die eine qualitative Aussage für jede produzierte Flasche (Behälter) erlaubt. Bei der oben beschriebenen Qualitätsmessung im Bereich der steigenden Druckkurve ist man von der Pumpenleistung der Evakuierungsphase unabhängig. Die eingangs beschriebene, genauere Qualitätsmessung wird deshalb durch die Vor- oder Indikationsmessung nicht ersetzt. Sie ergänzt diese nur, denn man kann aus dem Vormessen sehr früh Rückschlüsse auf den Beschichtungsprozess ziehen und diesen entsprechend steuern.

Zur Durchführung des Verfahrens nach einem der vorstehend diskutierten Ausführungsformen ist erfindungsgemäß vorgesehen, dass ein erstes Messrohr aus dem Innenraum des Behälters durch einen Stopfen nach außerhalb des abschließbaren Gefäßes geführt und mit einem Druckmessgerät sowie über wenigstens ein Ventil mit einer an eine Vakuumpumpe angeschlossenen Gefäßentleerungsleitung verbunden ist und dass zwischen der Anschlussstelle der Gefäßentleerungsleitung an das abschließbare Gefäß und im ersten Messrohr ein Messventil und ein fein regulierbares Ventil in Serie geschaltet sind. Als Behälter kann man sich für die eigene Vorstellung eine Kunststoffflasche mit einem Stopfen denken, durch welchen ein erstes Messrohr geführt ist. Auf diese Weise hat man Zugang zu dem Innenraum im Behälter, kann diesen evakuieren und den darin herrschenden Druck messen. Letzteres erfolgt über ein angeschlossenes Druckmessgerät, welches sich außerhalb des abschließbaren Gefäßes befindet. Das erste Messrohr kann aus dem Innenraum des Behälters und auch dem Innenraum des abschließbaren Gefäßes nach außerhalb geführt werden, wo sich das Druckmessgerät befindet.

Ferner ist erfindungsgemäß bei der hier betrachteten Vorrichtung eine Vakuumpumpe angeschlossen, die über wenigstens ein Ventil mit dem ersten Messrohr in Verbindung steht. Weil man außerdem das abschließbare Gefäß ebenso evakuieren will, ist an dieses Gefäß eine Gefäßentleerungsleitung angeschlossen, die ebenfalls mit der Vakuumpumpe über Verbindungsleitungen gekoppelt ist. Dieser Aufbau ist einfach und gestattet einfache Messgeräte sowie eine Qualitätsbestimmung der zu messenden Barriereschicht in kurzer Zeit, vorzugsweise weniger als 5 Minuten. Wenn man

(Es folgt der Rest der ursprünglichen Beschreibung, beginnend ab Seite 9 oben bis zum Ende der Beschreibung unverändert.) eine besonders einfache Kapazitätsmeßröhre zur Druckmessung verwendet, kann man die erwähnten Vorteile bereits erreichen, wobei insbesondere an die Summe aller Partialdrücke der aus der Behälterwandung abgebebenen Stoffe gedacht ist.

Wenn bei einer anderen Ausführungsform der Vorrichtung das Druckmeßgerät ein Massenspektrometer ist, welches über ein Schutzventil mit dem ersten Meßrohr verbunden ist, kann man den Druckaufbau für einzelne aus der Behälterwandung abgegebene Stoffe messen. Man hat zum Beispiel überraschend festgestellt, daß ein mit Hilfe des Massenspektrometers gemessener Stoff mit u = 17 atomaren Masseneinheiten mit dem späteren Auftreten des bekannten, bereits oben erwähnten Kunststoffgeschmacks im eingefüllten Behältergut (Wasser in einer Sprudelflasche) korreliert sein kann. Mit einem Massenspektrometer kann man dann auch schnell und einfach das erfindungsgemäße Verfahren hinsichtlich geschmacksrelevanter Stoffe durchführen.

Wenn bei einer weiteren Ausgestaltung der Erfindung wenigstens das erste Meßrohr kurz ist und aus nicht-rostendem Stahl besteht, erhält man weniger Meßverfälschungen, denn aus dem Material des nicht-rostenden Stahls können nur wenige Stoffe abdampfen und einen Beitrag bei der Druckmessung liefern. Deshalb ist man bestrebt, das erste Meßrohr und die notwendigerweise damit verbundenen weiteren Rohre möglichst kurz auszugestalten.

Erfindungsgemäß kann man weiterhin zwischen der Anschlußstelle der Gefäßentleerungsleitung an das abschließbare Gefäß und dem ersten Meßrohr ein Meßventil und ein fein regulierbares Ventil, wie zum Beispiel ein Nadelventil, in Serie schalten. Während das Meßventil erforderlich ist, um die Messung zu ermöglichen (wenn das Meßventil geschlossen ist), erlaubt der Einsatz des Nadelventils mit Vorteil den Einsatz nur einer einzigen Vakuumpumpe. Mit dieser Pumpe kann man dann das abschließbare Gefäß einerseits (Außenraum um den Behälter) als auch den Innenraum des zu messenden Behälters mit eben dieser einen Vakuumpumpe evakuieren. Das Nadelventil erlaubt nämlich die Regelung des Gasflusses beim Abpumpen; d.h. Regelung des Gasflusses aus dem zu messenden Behälter in Relation zu dem Gasfluß aus dem abschließbaren Gefäß, in welchem der Behälter angeordnet ist. Die Drücke innerhalb und außerhalb des zu messenden Behälters sollen nämlich zweckmäßigerweise ähnlich sein. Große Druckunterschiede sind zu vermeiden. Wäre der Druck außerhalb des Behälters erheblich größer als innerhalb, wäre verständlicherweise ein Kollabieren des Behälters zu befürchten; umgekehrt könnte der Stopfen in dem zu messenden Behälter aus dessen Öffnung herausgedrückt oder gar der Behälter selbst zum Platzen gebracht werden, wenn der Druck außerhalb des Behälters, nämlich innerhalb des abzuschließenden Gefäßes, erheblich kleiner (eine Größenordnung Unterschied) als der Innendruck im Behälter wäre.

Weitere Vorteile, Merkmale und Anwendungsmöglichkeiten der vorliegenden Erfindung ergeben sich aus der folgenden Beschreibung bevorzugter Ausführungsbeispiele der Erfindung in Verbindung mit den Zeichnungen. In diesen zeigen:
- Figur 1: schematisch eine erste Vorrichtung zur Durchführung des erfindungsgemäßen Bestim- mungsverfahrens,
- Figur 2: eine ähnliche Vorrichtung wie Figur 1, jedoch unter Einsatz eines Massenspektrometers zur Messung der Druck-Zeit-Kurve bzw. Intensitäts-Zeit-Kurve,
- Figur 3: die Druck-Zeit-Kurve für unterschiedliche, aus der Behälterwandung entgaste Stoffe,
- Figur 4a: schematisch die Meßdarstellung in einem Massenspektrometer mit Linien für unterschied- liche atomare Masseneinheiten,
- Figur 4b: eine Intensitäts-Zeit-Kurve für zwei ausgewählte Gase, die in dem Massenspektrometer der Figur 2 gemessen wurden, und
- Figur 5: eine ähnliche Druck-Zeit-Kurve wie nach Figur 3, wobei allerdings auch der anfängliche Entgasungsbereich dargestellt ist.

Der zu messende Behälter 1 ist als Wasserflasche aus Kunststoff ohne Füllgut (Wasser) gezeigt und in seinem Flaschenhals mit einem Stopfen 2 verschlossen, durch welchen ein erstes Meßrohr 3 hindurchgeführt ist. Das untere offene Ende 4 des ersten Meßrohres 3 endet etwa in der Mitte des Behälters 1, der sich in einem druckdicht abschließbaren Gefäß 5 im Abstand von dessen Wandungen befindet. Das erste Meßrohr 3 führt aus dem Innenraum 6 des Behälters 1 durch den Stopfen 2 und durch die Oberwand des abschließbaren Gefäßes 5 hindurch nach oben, um an dem Anschluß 7 des als Kapazitätsmeßröhre ausgestalteten Druckmeßgerätes 8 zu enden.

Über ein Abgangsventil 9 ist eine Vakuumpumpe 10 an einer Ringleitung 11 angeschlossen, mit deren Hilfe das abschließbare Gefäß 5 mit Hilfe der Gefäßentleerungsleitung 12 evakuiert werden kann. Zu diesem Zweck ist eine Anschlußstelle 13 an dem abschließbaren Gefäß 5 vorgesehen, durch welche die Gefäßentleerungsleitung 12 mit dem Gefäß 5 einerseits und der Ringleitung 11 andererseits in direkter Verbindung steht, so daß bei geöffnetem Ventil 9 und eingeschalteter Vakuumpumpe 10 der Innenraum 14 des abschließbaren Gefäßes 5 evakuiert werden kann.

In der Außenwandung des abschließbaren Gefäßes 5 gibt es noch eine weitere Anschlußstelle 15, die mit einer Belüftungsleitung 16 sowie Meß- (17) als auch Saugleitung (18) verbunden ist. Über ein Hilfsventil 19 kann die Saugleitung 18 mit der Ringleitung 11 verbunden werden. Ein Belüftungsventil 20 gestattet beim Öffnen das Belüften des Innenraums 14 des Gefäßes 5. An der Meßleitung 17 ist ein einfaches Druckmeßgerät 21 angeschlossen, das im vorliegenden Aufbau mit einer Pirani-Röhre arbeitet.

Schließlich verbindet die Verbindungsleitung 22 das erste Meßrohr 3 über zwei Ventile mit der Ringleitung 11. Bei diesen zwei in Reihe geschalteten Ventilen handelt es sich um ein Meßventil 23 und ein Nadelventil 24. An dem Verbindungspunkt 25 laufen die Ringleitung 11, die Gefäßentleerungsleitung 12 und die mit den beiden Ventilen 24 und 23 bestückte Verbindungsleitung 22 zusammen.

Im Betrieb werden bei geschlossenem Belüftungsventil 20 und geöffneten Ventilen 19, 23, 24 und 9 der Behälter 1 über das erste Meßrohr 3 wie auch das abschließbare Gefäß 5 evakuiert. Es entsteht zwischen dem Innenraum 6 des Behälters 1 und dem Innenraum 14 des Gefäßes 5 keine große Druckdifferenz. Der Druck im Innenraum 14 des Gefäßes 5 wird über das Druckmeßgerät 21 gemessen. Das Nadelventil 24 sorgt für die Regelung der Gasflüsse aus dem Innenraum 6 bezüglich derer aus dem Innenraum 14.

Nach Schließen des Meßventils 23 und vorzugsweise auch der Ventile 9 und 19 wird der Druck im Innenraum 6 des Behälters 1 über die Kapazitätsmeßröhre 8 gemessen.

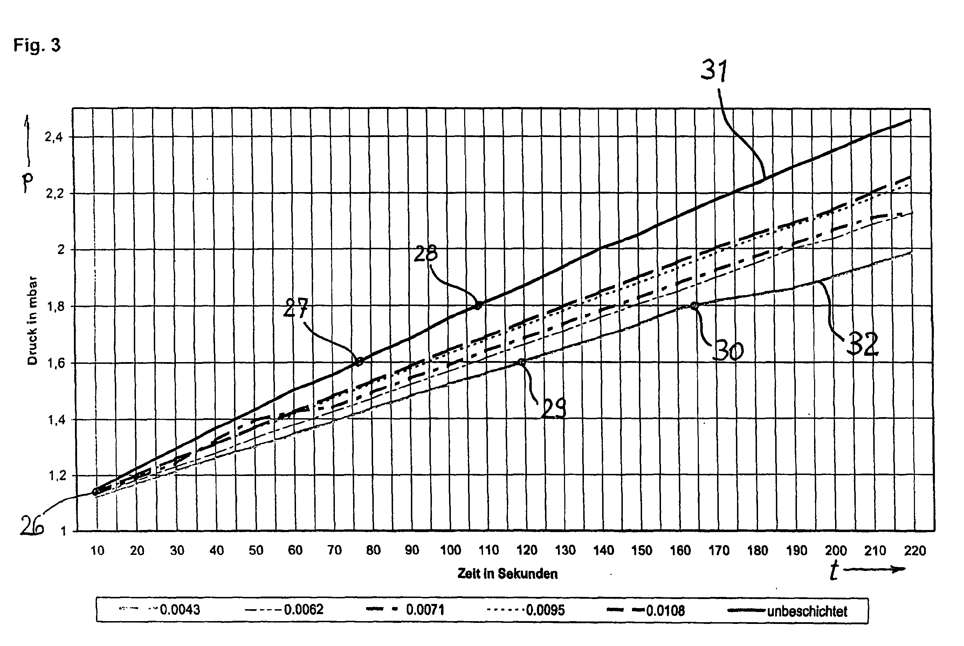

Betrachtet man Figur 3, dann geht man praktischerweise bei diesem Ausführungsbeispiel davon aus, daß zur Zeit t = 10 Sekunden ein Druck von etwa 1,15 mbar (erster Meßpunkt 26) gemessen wird. Die obere Kurve 31 stellt die Druck-Zeit-Kurve einer nicht beschichteten Flasche/eines Behälters dar.

Nach Schließen des Meßventils 23 hat das als Kapazitätsmeßröhre ausgebildete Druckmeßgerät 8 nach 77 Sekunden mit dem zweiten Meßpunkt 27 einen Druckwert von 1,6 mbar erreicht. Unter der Annahme, daß ab hier in einem gewissen Bereich eine lineare Abhängigkeit zwischen Druck und Zeit vorliegt, wird 31 Sekunden lang gewartet und dann bei Erreichen des Druckes von 1,8 mbar mit dem dritten Meßpunkt 28 eine Zeit von 120 Sekunden gemessen. Die Steigung dieser Kurve (Geraden) erlaubt die Aussage, daß die Qualität der Barriereschicht mangelhaft ist. Die Kurve 31 stellt den nicht beschichteten Behälter dar.

Im Vergleich dazu stellt die untere Kurve 32 das Druck-Zeit-Verhältnis eines mit einer guten Barriereschicht versehenden Behälters 1 dar. Zwischen dem vierten Meßpunkt 29, bei welchem der Anfangsdruck von 1,6 mbar erreicht ist, und dem fünften Meßpunkt 30, bei welchem der ausgesuchte Endpunkt von 1,8 mbar erreicht ist, liegt eine Zeitdifferenz von 45 Sekunden mit der Aussage, daß diese geringere Steigung für gute Barriereeigenschaften spricht.

Nach einer solchen Messung, die etwa 4 Minuten dauerte, schließt man die Ventile 9 und 19 und belüftet den Innenraum 14 des Gefäßes 5 über das geöffnete Belüftungsventil 20.

Danach wiederholt sich die Messung für den nächsten Behälter 1.

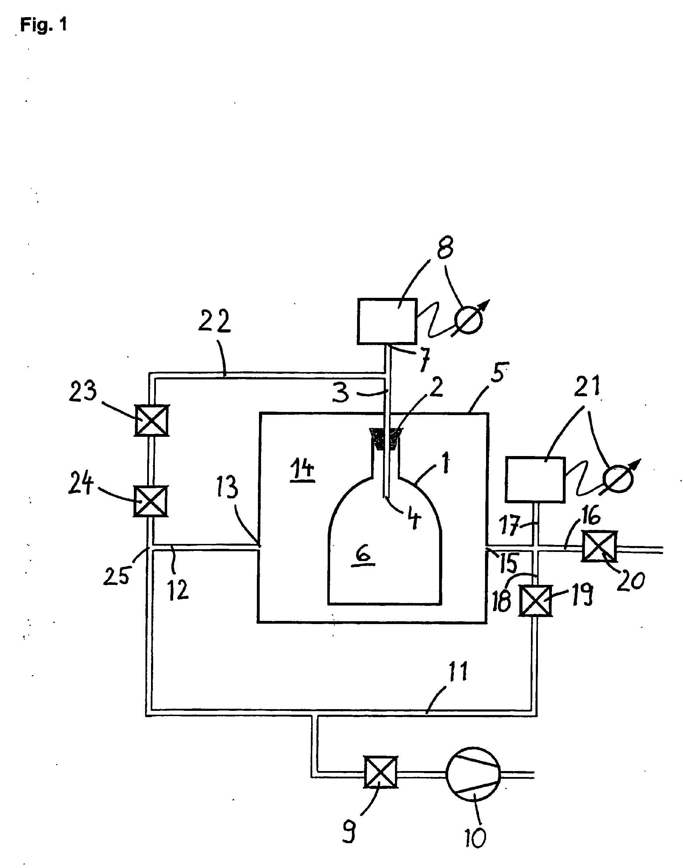

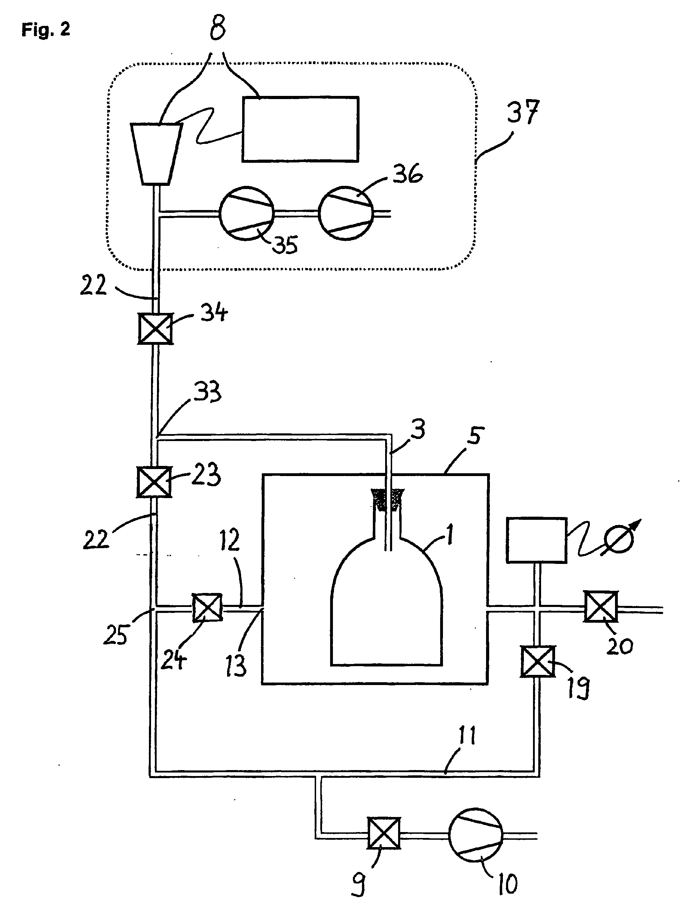

Die Vorrichtung der Figur 2 unterscheidet sich von der der Figur 1 in nur zweierlei Hinsicht:
1. Der erste Unterschied liegt darin, daß das erste Meßrohr 3 an einer weiteren Anschlußstelle 33 an die Verbindungsleitung 22 angeschlossen ist, die über ein Schutzventil 34 die Verbindung zu dem als Massenspektrometer ausgebildeten Druckmeßgerät 8 herstellt. Eine Turbomolekularpumpe 35 mit nachgeschalteter Vorpumpe 36 sorgt für eine Reduzierung des Rohrinnendruckes in der Verbindungsleitung 22 zwischen Schutzventil 34 und dem Massenspektrometer 8.
2. Betrachtet man die Verbindungsleitung 22 von der Anschlußstelle 13 an dem abzuschließenden Gefäß 5 und der weiteren Anschlußstelle 33 zwischen dem Schutzventil 34 und dem Meßventil 23, dann liegen zwar das Meßventil 23 und das Nadelventil 24 wieder in Reihe. Das Nadelventil 24 liegt aber in der Gefäßentleerungsleitung 12 zwischen der Anschlußstelle 13 und dem Verbindungspunkt 25. Auch in dieser Position hat das Nadelventil die gleiche Funktion und Zielsetzung wie oben erwähnt, auch in Verbindung mit Figur 1.

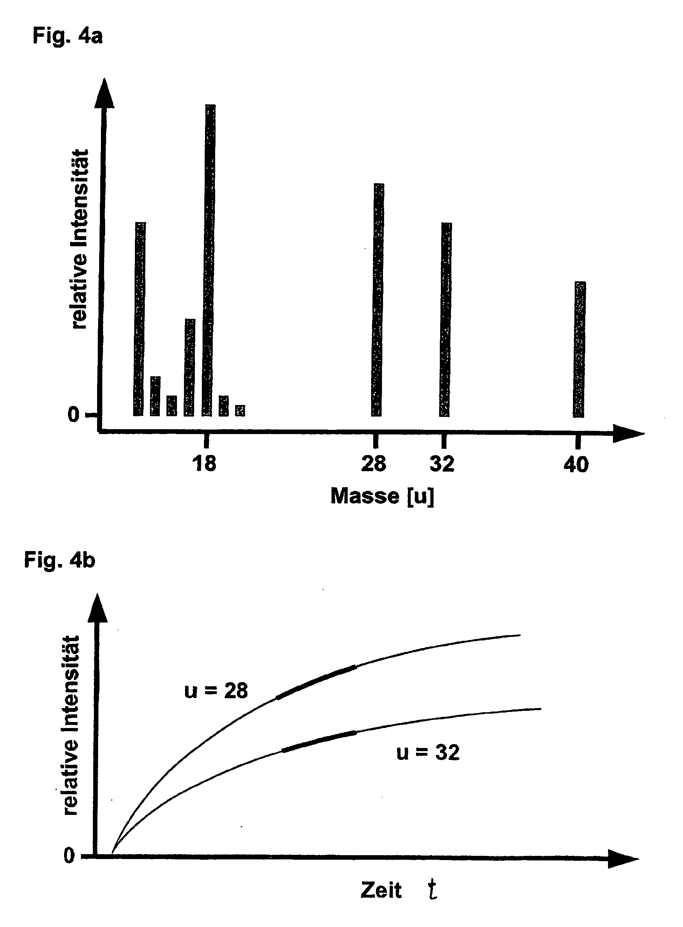

Auf dem Display des Massenspektrometers 8 sieht man die einzelnen Peaks mit den verschiedenen atomaren Masseneinheiten gemäß Figur 4a. Hier sind Peaks eingezeichnet bei u (X-Achse) zum Beispiel gleich14, 18, 28, 32, 40. Die nicht näher bezeichneten Peaks entsprechen zum Beispiel unter anderem dem Wasser und wachsen im Verlauf der Messung über der Zeit langsam an, wie zum Beispiel die Darstellung der Figur 4b zeigt. Auf dieser ist über der Zeit t die relative Peakintensität aufgetragen. Für u = 28 ist eine Kurve (oben) und für u = 32 ist eine weitere Kurve (unten) gezeigt. Beide haben den betont herausgezeichneten linearen Bereich, so daß aus der Steigung dieser Kurve die Qualität der Barriere für den Stoff mit u = 28 usw. bestimmt werden kann.

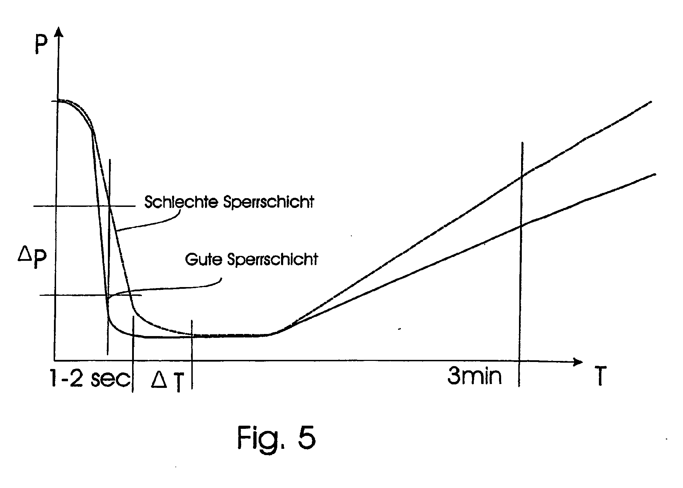

Figur 5 zeigt eine ähnliche Druck-Zeit-Kurve wie Figur 3. In Figur 5 ist über der Zeit T wieder der Druck p aufgetragen. Dabei werden zwei Kurven betrachtet: Die in durchgezogener Linie dargestellte Kurve ist die für eine gute Sperrschicht und die mit gestrichelter Linie gezeigte Kurve die mit schlechter Sperrschicht. Die im rechten Teil gezeigten geraden Äste entsprechen der Kurve der Figur 3. Das eigentliche Qualitätsmessen erfolgt also nur in diesem Bereich, nachdem evakuiert wurde und der untere horizontale Niveaubereich der beiden Kurven der Figur 5 erreicht ist.

In Figur 5 ist nun zusätzlich auch links der anfängliche Entgasungsbereich gezeigt. Man kann bei der Durchführung des Verfahrens vom Nullpunkt ausgehen und verringert beim Entgasen oder Evakuieren den Druck. Gelingt diese Druckverringerung nur langsamer entsprechend der gestrichelten Kurve, dann hat man es mit der schlechten Sperrschicht zu tun. Die schlechte Sperrschicht ist nämlich nicht so gut in der Lage, in der Wandung eingeladene Stoffe, z.B. Gase, zurückzuhalten.

Entsprechend steiler verläuft die Kurve, die mit durchgezogenen Linien dargestellt ist und die eine gute Sperrschicht veranschaulicht. Diese Sperrschicht läßt den Druck auf der Seite mit der Beschichtung schneller abfallen, weil nicht so viele eingeladene Gase durch die Schicht in den Raum, der durch die Schicht begrenzt ist, eintreten. Bei dem hier gezeigten Beispiel ist ein bestimmter unterer Wert des Druckbereiches Δp nach 1 bis 2 Sekunden erreicht. Der obere Druckgrenzpunkt wird sogar kurz vorher schon erreicht. Nimmt man die Ausführungsform, bei welcher die Partialdrücke nach einer festen Zeit gemessen werden, dann muß man feststellen, ob die gemessenen Partialdrücke in dem erlaubten Druckbereich Δp liegen, z.B. über dessen unterem Grenzwert liegen.

Wenn man andererseits einen vorgegebenen Zielpartialdruck erreichen will, dann kann nach der zweiten Ausführungsform diejenige Zeit gemessen werden, in welcher der Zieldruck erreicht wird. Hier ist auf der Abszisse die Zeit ΔT eingetragen. Erreicht man z.B. den unteren Grenzwert des Druckes innerhalb des Zeitintervalls ΔT oder vorher, dann hat man eine gute Sperrschicht gemessen.

Die Qualitätsmessung der produzierten Behälter wird unbeachtlich dieser Vor- oder Indikationsmessung in einer Zeit von etwa 3 Minuten ab Beginn des Evakuierens gemessen.

### Bezugszeichenliste:

- 1: Behälter
- 2: Stopfen
- 3: erstes Meßrohr
- 4: unteres, offenes Ende des Meßrohres
- 5: druckdicht abschließbares Gefäß
- 6: Innenraum des Behälters
- 7: Anschluß des Druckmeßgerätes
- 8: Druckmeßgerät, bestehend aus schematisch gezeichneter Meßröhre und Anzeigegerät
- 9: Abgangsventil
- 10: Vakuumpumpe
- 11: Ringleitung
- 12: Gefäßentleerungsleitung
- 13: Anschlußstelle
- 14: Innenraum des abschließbaren Gefäßes
- 15: weitere Anschlußstelle
- 16: Belüftungsleitung
- 17: Meßleitung
- 18: Saugleitung
- 19: Hilfsventil
- 20: Belüftungsventil
- 21: Druckmeßgerät, bestehend aus schematisch gezeichneter Pirani-Meßröhre und Anzeigege- rät
- 22: Verbindungsleitung
- 23: Meßventil
- 24: feinregulierbares Ventil
- 25: Verbindungspunkt
- 26: erster Meßpunkt
- 27: zweiter Meßpunkt
- 28: dritter Meßpunkt
- 29: vierter Meßpunkt
- 30: fünfter Meßpunkt
- 31: obere Kurve (unbeschichtet)
- 32: untere Kurve (mit guter Barriere)
- 33: weitere Anschlußstelle
- 34: Schutzventil
- 35: Turbomolekularpumpe
- 36: Vorpumpe
- 37: Gesamtheit Massenspektrometer

## Patentansprüche

1. Verfahren zum Bestimmen der Permeation einer Barriereschicht auf der Oberfläche eines Behälters (1), bei welchem der Behälter (1) in ein druckdicht abschließbares Gefäß (5) gebracht wird, der Druck über der Zeit gemessen, die Steigung der Druck-Zeit-Kurve ermittelt wird und aus diesen Messdaten die Permeation der Barriereschicht bestimmt wird,
**dadurch gekennzeichnet, dass**
- das druckdicht abschließbare Gefäß (5) und der darin befindliche Behälter (1) evakuiert werden,
- nach einer Konditionierungszeit der Behälter (1) auf der Seite mit der Barriereschicht entgasen gelassen wird und
- der Partialdruck wenigstens eines aus der Behälterwandung abgegebenen Stoffes, insbesondere Gases, gemessen wird.

2. Verfahren nach Anspruch 1, **dadurch gekennzeichnet, dass** nach dem Evakuieren die Summe der Partialdrücke aller aus der Behälterwandung abgegebenen Stoffe gemessen wird.

3. Verfahren nach Anspruch 1 oder 2, **dadurch gekennzeichnet, dass** der Partialdruck wenigstens eines von der Behälterwandung abgegebenen, für die geschmackliche Beeinflussung der Behälterfüllung relevanten Stoffes gemessen wird.

4. Verfahren nach einem der Ansprüche 1 bis 3, **dadurch gekennzeichnet, dass** die Qualität der Barriereschicht eines Referenzbehälters zum Eichen der Steigung der gemessenen Druck-Zeit-Kurve verwendet wird.

5. Verfahren nach einem der Ansprüche 1 bis 4, **dadurch gekennzeichnet, dass** in der Konditionierungszeit alle dem Bestimmungsverfahren zu unterziehenden Behälter (1) gleichmäßig mit Molekülen beladen werden.

6. Verfahren nach einem der Ansprüche 1 bis 5, **dadurch gekennzeichnet, dass** der Behälter (1) und das druckdicht abschließbare Gefäß (5) in etwa einer Minute evakuiert werden.

7. Verfahren nach einem der Ansprüche 1 bis 6, **dadurch gekennzeichnet, dass** das Entgasen und Messen des wenigstens eines Partialdruckes insgesamt weniger als 8 Minuten, vorzugsweise 6 Minuten, und ganz besonders bevorzugt etwa 4 Minuten dauern.

8. Verfahren nach Anspruch 1 oder 2, **dadurch gekennzeichnet, dass** bei dem Evakuieren die Summe aller Partialdrücke des im Behälter (1) oder außerhalb desselben befindlichen Gases und der aus der Behälterwandung abgegebenen Stoffe gemessen werden.

9. Verfahren nach Anspruch 8, **dadurch gekennzeichnet, dass** die Partialdrücke nach einer festgesetzten Zeit gemessen und mit einem Zieldruckwert (Δp) verglichen werden.

10. Verfahren nach Anspruch 8, **dadurch gekennzeichnet, dass** die Zeit gemessen wird, in der ein vorgegebener Zielpartialdruck erreicht wird.

11. Vorrichtung zur Durchführung des Verfahrens nach einem der Ansprüche 1 bis 7,
**dadurch gekennzeichnet, dass** ein erstes Messrohr (3) aus dem Innenraum (6) des Behälters (1) durch einen Stopfen (2) nach außerhalb des abschließbaren Gefäßes (5) geführt und mit einem Druckmessgerät (8) sowie über wenigstens ein Ventil (23, 24, 9) mit einer an eine Vakuumpumpe (10) angeschlossenen Gefäßentleerungsleitung (12) verbunden ist und dass zwischen der Anschlussstelle (13) der Gefäßentleerung (12) an das abschließbare Gefäß (5) und dem ersten Messrohr (3) ein Messventil (23) und ein feinregulierbares Ventil (24) in Serie geschaltet sind.

12. Vorrichtung nach Anspruch 11, **dadurch gekennzeichnet, dass** das Druckmessgerät (8) eine Kapazitätsmessröhre ist.

13. Vorrichtung nach Anspruch 11, **dadurch gekennzeichnet, dass** das Druckmessgerät (8) ein Massenspektrometer ist, welches über ein Schutzventil (34) mit dem ersten Messrohr (3) verbunden ist.

14. Vorrichtung nach einem der Ansprüche 11 bis 13, **dadurch gekennzeichnet, dass** wenigstens das erste Messrohr (3) kurz ist und aus nicht-rostendem Stahl besteht.

## Claims

1. A method for determining the permeation of a barrier layer on the surface of a container (1), in which the container (1) is put into a pressure-tightly closable vessel (5), the pressure in relation to time is measured, the gradient of the pressure-time curve is determined and permeation of the barrier layer is determined from those measurement data, **characterized in that**
- the pressure-tightly closable vessel (5) and the container (1) disposed therein are evacuated;
- after a conditioning time, the container (1) is left to degas on the side with the barrier layer;
- the partial pressure of at least one substance emitted from the container wall, particularly gas, is measured.

2. A method as set forth in claim 1, **characterised in that** after evacuation, the sum of all partial pressures of all substances emitted from the container wall are measured.

3. A method as set forth in claim 1 or 2, **characterised in that** the partial pressure of at least one substance which is emitted from the container wall and which is relevant in terms of the container filling being influenced in its taste is measured.

4. A method as set forth in one of the claims 1 to 3, **characterised in that** the quality of the barrier layer of a reference container is used for calibrating the gradient of the measured pressure-time curve.

5. A method as set forth in one of the claims 1 to 4, **characterised in that** in the conditioning time all containers (1) to be subjected to the determination method are uniformly loaded with molecules.

6. A method as set forth in one of the claims 1 to 5, **characterised in that** the container (1) and the pressure-tightly closable vessel (5) are evacuated in about one minute.

7. A method as set forth in one of the claims 1 to 6, **characterised in that** degassing and measurement of the at least one partial pressure lasts in total less than 8 minutes, preferably 6 minutes and quite particularly preferably about 4 minutes.

8. A method as set forth in claim 1 or 2, **characterised in that** in the evacuation step the sum of all partial pressures of the gas in the container (1) or outside same and the substances emitted from the container wall are measured.

9. A method as set forth in claim 8, **characterised in that** the partial pressures are measured after an established time and compared to a target pressure value (Δp).

10. A method as set forth in claim 8, **characterised in that** the time in which a predetermined target partial pressure is reached is measured.

11. Apparatus for carrying out the method as set forth in one of claims 1 through 7, **characterised in that** a first measuring tube (3) is passed out of the internal space (6) of the container (1) through a stopper (2) to outside the closable vessel (5) and connected to a pressure measuring device (8) and by way of at least one valve (23, 24, 9) to a vessel emptying conduit (12) connected to a vacuum pump (10) and **in that** a measuring valve (23) and a finely regulatable valve (24) are connected in series between the connection location (13) of the vessel emptying conduit (12) to the closable vessel (5) and the first measuring tube (3).

12. Apparatus as set forth in claim 11, **characterised in that** the pressure measuring device (8) is a capacitance measuring tube.

13. Apparatus as set forth in claim 11, **characterised in that** the pressure measuring device (8) is a mass spectrometer which is connected to the first measuring tube (3) by way of a protective valve (34).

14. Apparatus as set forth in one of the claims 11 through 13, **characterised in that** at least the first measuring tube (3) is short and comprises stainless steel.

## Revendications

1. Procédé pour la détermination de la perméation d'une couche barrière à la surface d'un récipient (1), procédé dans lequel on place le récipient (1) dans une cuve (5), pouvant être fermée de façon étanche à la pression, on mesure la pression dans le temps, on détermine la pente de la courbe pression-temps et on détermine la perméation de la couche barrière à partir de ces données mesurées,
**caractérisé en ce que**
- on fait le vide dans la cuve (5), pouvant être fermée de façon étanche à la pression, et dans le récipient (1) se trouvant à l'intérieur,
- après un temps de conditionnement, on fait dégazer le récipient (1) sur le côté comportant la couche barrière et
- on mesure la pression partielle d'au moins une substance relarguée par la paroi du récipient, en particulier d'un gaz.

2. Procédé selon la revendication 1, **caractérisé en ce que**, après l'obtention du vide, on mesure la somme des pressions partielles de toutes les substances relarguées par la paroi du récipient.

3. Procédé selon la revendication 1 ou la revendication 2, **caractérisé en ce qu'**on mesure la pression partielle d'au moins une substance relarguée par la paroi du récipient et importante pour agir sur le goût du produit à l'intérieur du récipient.

4. Procédé selon l'une quelconque des revendications 1 à 3, **caractérisé en ce qu'**on utilise la qualité de la couche barrière d'un récipient de référence pour étalonner la pente de la courbe pression-temps mesurée.

5. Procédé selon l'une quelconque des revendications 1 à 4, **caractérisé en ce que**, pendant le temps de conditionnement, on charge tous les récipients (1), à soumettre au procédé de détermination, de manière homogène avec des molécules.

6. Procédé selon l'une quelconque des revendications 1 à 5, **caractérisé en ce qu'**on fait le vide dans le récipient (1) et la cuve (5), pouvant être fermée de façon étanche à la pression, en une minute environ.

7. Procédé selon l'une quelconque des revendications 1 à 6, **caractérisé en ce que** le dégazage et la mesure d'au moins une pression partielle durent au total moins de 8 minutes, de préférence 6 minutes et de manière tout particulièrement préférée, environ 4 minutes.

8. Procédé selon la revendication 1 ou la revendication 2, **caractérisé en ce que**, lors de la mise sous vide, on mesure la somme de toutes les pressions partielles du gaz se trouvant dans le récipient (1) ou à l'extérieur de celui-ci et des substances relarguées par la paroi du récipient.

9. Procédé selon la revendication 8, **caractérisé en ce qu'**on mesure les pressions partielles à l'issue d'un laps de temps fixé et on les compare à une valeur de pression cible (Δρ).

10. Procédé selon la revendication 8, **caractérisé en ce qu'**on mesure le laps de temps pendant lequel une pression partielle cible prédéfinie est obtenue.

11. Dispositif pour la réalisation du procédé selon l'une quelconque des revendications 1 à 7,
**caractérisé en ce que**
on fait sortir un premier tube de mesure (3) de l'intérieur (6) du récipient (1), on le fait passer à travers un bouchon (2) vers l'extérieur de la cuve (5) pouvant être fermée et on le relie à un manomètre (8) ainsi qu'à un conduit d'évacuation (12) de la cuve, relié à une pompe à vide (10), par l'intermédiaire d'au moins une soupape (23, 24, 9), et on branche en série une soupape de mesure (23) et une soupape de précision (24) entre le point de raccordement (13) de l'évacuation (12) à la cuve (5), pouvant être fermée, et le premier tube de mesure (3).

12. Dispositif selon la revendication 11, **caractérisé en ce que** le manomètre (8) est un capacimètre.

13. Dispositif selon la revendication 11, **caractérisé en ce que** le manomètre (8) est un spectromètre de masse lié au premier tube de mesure (3) par une soupape de protection (34).

14. Dispositif selon l'une quelconque des revendications 11 à 13, **caractérisé en ce qu'**au moins le premier tube de mesure (3) est court et en acier inoxydable.
